# EUROPÄISCHE PATENTSCHRIFT

(11) **EP 3 518 800 B1**
(45) Veröffentlichungstag und Bekanntmachung des Hinweises auf die Patenterteilung: **13.04.2022**
(21) Anmeldenummer: 17784699.5
(22) Anmeldetag: 29.09.2017
(51) Int. Cl.: A61B 17/92, A61B 17/00

(54) **SCHLAGWERKZEUG ZUR KNOCHENBEARBEITUNG BEI HÜFTOPERATIONEN**
STRIKING TOOL FOR TREATMENT OF BONES IN HIP OPERATIONS
OUTIL À PERCUSSION POUR L'USINAGE D'OS LORS D'OPÉRATIONS DE LA HANCHE

(30) Priorität: 03.10.2016 CH 13052016
(43) Veröffentlichungstag der Anmeldung: 07.08.2019
(73) Patentinhaber: IBAG Medtech AG, 8302 Kloten (CH)
(72) Erfinder: BUFF, Dominic, 8302 Kloten (CH); KALBERER, Fabian, 8123 Ebmatingen / ZH (CH); NASIC, Milos, 8052 Zürich (CH); WALDVOGEL, Daniel, 8425 Oberembrach (CH)
(74) Vertreter: Kieffer, Valentin
(86) Internationale Anmeldenummer: PCT/IB2017/056019
(87) Internationale Veröffentlichungsnummer: WO 2018/065873

(56) Entgegenhaltungen:
- DE-A1-102013 016 171

## Beschreibung

Die vorliegende Erfindung betrifft ein Schlagwerkzeug zur Knochenbearbeitung bei Hüftoperationen gemäss Oberbegriff des Patentanspruchs 1.

Bereits seit vielen Jahren werden bei Hüftoperationen pneumatische Schlagwerkzeuge unterstützend eingesetzt. Insbesondere für die Bearbeitung eines Knochens zur Aufnahme des künstlichen Gelenkteils ist ein solches Schlagwerkzeug von Vorteil, da die Belastung des Knochens mit einer optimal abgestimmten Maschine gegenüber der Ausführung von Hand minimiert werden kann. Solche Schlagwerkzeuge sind aus EP 0 452 543 und EP 0 617 926 bekannt.

Seit einigen Jahren wird als Alternative zur herkömmlichen Methode der Hüftoperation die AMIS-Methode (Anterior Minimal Invasive Surgery) angeboten. Insbesondere aufgrund der kürzeren Rehabilitationszeit nach der Operation, wird diese Operationsmethode immer häufiger durchgeführt. Bei der AMIS-Methode liegt der Patient auf dem Rücken, so dass der Operateur von Oben den Knochen für die Aufnahme des künstlichen Gelenkteils vorbereiten muss. Die bekannten pneumatischen Schlagwerkzeuge könnten zwar grundsätzlich auch bei dieser Operationsmethode zum Einsatz kommen, allerdings sind sie unhandlich und aufgrund der Länge des Schlagwerkzeugs müsste sich der Operateur gegenüber dem Patienten auf einer erhöhten Plattform befinden um das Schlagwerkzeug richtig bedienen zu können. Daher wird die Knochenbearbeitung für Hüftoperationen gemäss der AMIS-Methode vom Operateur in Handarbeit durchgeführt.

Die vorliegende Erfindung stellt sich nunmehr die Aufgabe, ein Gerät der Eingangs genannten Art derart zu verbessern, dass die Vorteile der bekannten pneumatischen Schlagwerkzeuge erhalten bleiben, das Gerät aber handlicher ist und auch für Hüftoperationen gemäss AMIS-Methode eingesetzt werden kann. Ebenfalls stellt sich die vorliegende Erfindung die Aufgabe die Durchführung der AMIS-Methode mit Anwendung des vorgestellten Schlagwerkzeugs zu verbessern.

Diese Aufgabe löst ein Schlagwerkzeug für Hüftoperationen mit den Merkmalen des Patentanspruches 1. Weitere Merkmale und Ausführungsbeispiele gehen aus den abhängigen Ansprüchen hervor und deren Vorteile sind in der nachfolgenden Beschreibung erläutert.

In der Zeichnung zeigt:
- Figur 1a: Aufsicht des Schlagwerkzeugs
- Figur 1b: Seitenansicht des Schlagwerkzeugs
- Figur 1c: Vorderansicht des Schlagwerkzeugs
- Figur 2a-b: Neigung des Beins des Patienten für einen besseren Zugang zum Oberschenkelknochen
- Figur 2c: Arbeitsrichtung des Schlagwerkzeugs in einem Winkel zum Oberschenkelknochen
- Figur 2d: Seitenansicht des Schlagwerkzeugs mit einem Winkel α zwischen der Griffebene und der Längsachse ZK der Zylinder/Kolbenanordnung
- Figur 2e: Einsatz des Schlagwerkzeugs mit geneigten Griffen
- Figur 3: Aufsicht des Schlagwerkzeugs mit Griffe 3, deren Längsachse G in der Griffebene einen Winkel β aufweisen.

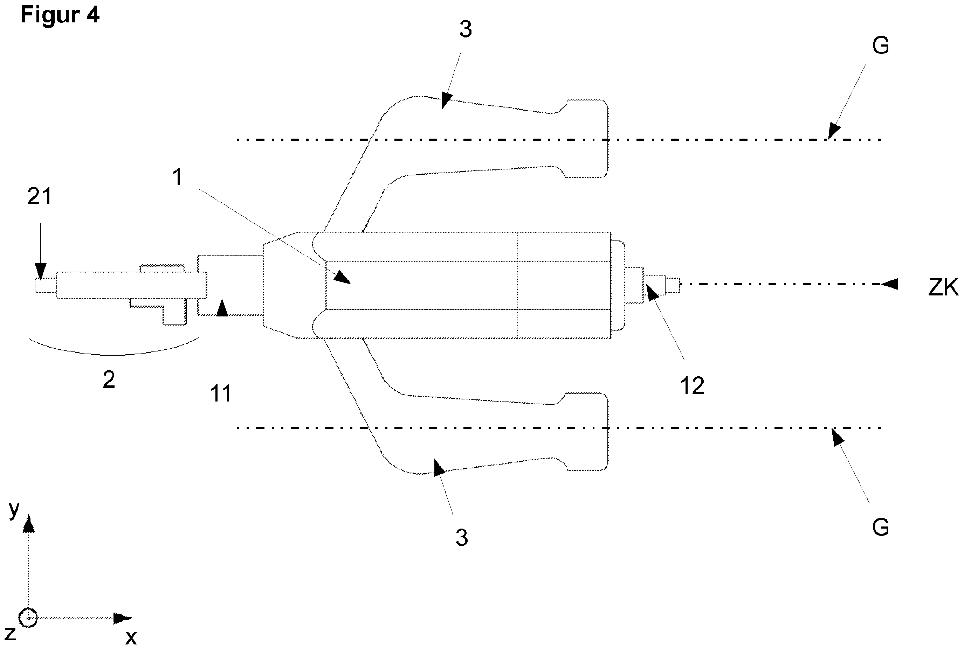

- Figur 4: Aufsicht des Schlagwerkzeugs mit Griffen, die nur an einem Ende mit dem Gehäuse 1 verbunden sind
- Figur 5: Seitenansicht des Schlagwerkzeugs mit geknicktem Werkzeughalter
- Figur 6: Aufsicht des Schlagwerkzeugs mit Werkzeughalter mit Versatz
- Figur 7a: Aufsicht des Schlagwerkzeugs mit abgewinkeltem Schlauchanschluss
- Figur 7b: Seitenansicht des Schlagwerkzeugs mit abgewinkeltem Schlauchanschluss
- Figur 7c: Seitenansicht des Schlagwerkzeugs mit seitlich angebrachtem Schlauchanschluss
- Figur 8: Aufsicht des Schlagwerkzeugs mit Schlauchanschluss mit drehbarem Kugelgelenk

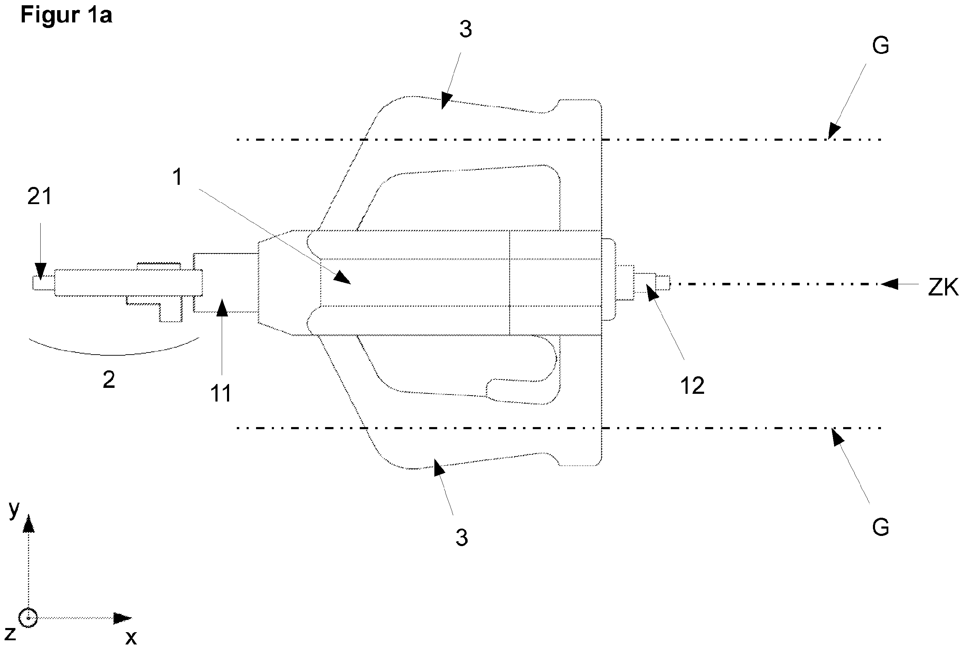

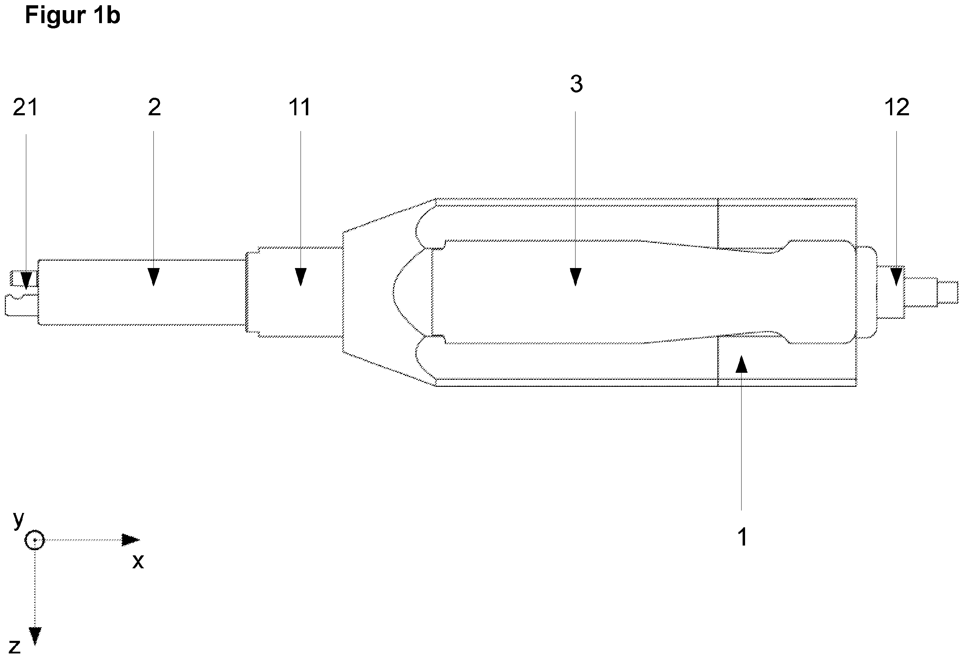

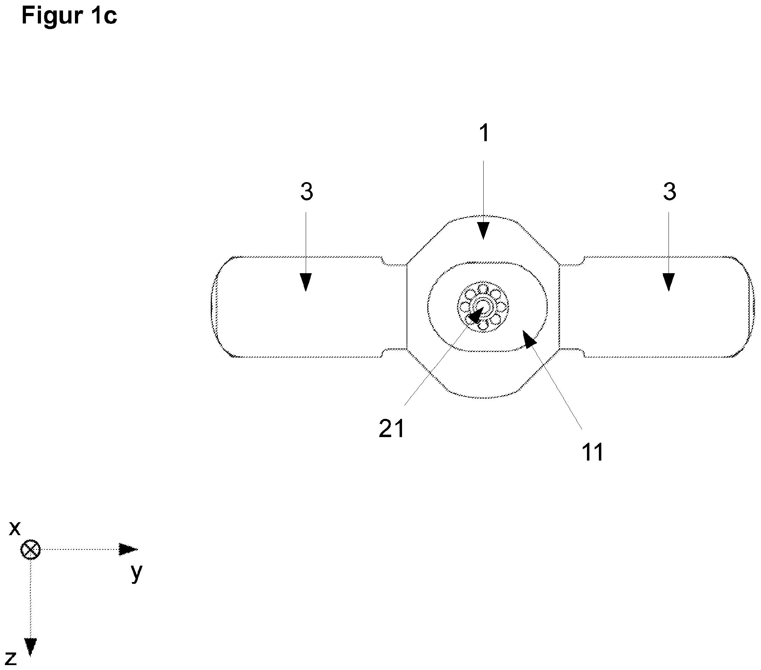

Die Figuren stellen mögliche Ausführungsbeispiele dar, welche in der nachfolgenden Beschreibung erläutert werden. Die Achsen x, y und z definieren ein orthogonales, rechtshändiges Koordinatensystem orientiert gemäss Figur 1.

Das vorliegende Schlagwerkzeug besteht aus einem Gehäuse **1**, mit welchem ein Werkzeughalter **2** und zwei oder mehr Griffe **3** verbunden sind (Figuren 1a-c). Innerhalb des Gehäuses **1** befindet sich eine Zylinder/Kolbenanordnung, wobei ein pneumatisch oder elektrisch angetriebener Kolben sich in einem Zylinder hin und her bewegt. Der Werkzeughalter **2** ist mit der Zylinder/Kolbenanordnung über einen Anschluss **11** derart verbunden, dass die Kräfte und/oder Bewegungen der Zylinder/Kolbenanordnung dem Werkzeughalter **2** wirksam übermittelt werden. In möglichen Ausführungsvarianten kann der Werkzeughalter **2** beispielsweise in oszillierender, vibrierender oder schlagender Bewegung angetrieben werden. Der Werkzeughalter **2** weist am vorderen Ende einen Anschluss **21** auf, an welchem verschiedene Werkzeuge zur Knochenbearbeitung befestigt werden können.

Für die Bearbeitung der Knochen zur Aufnahme des künstlichen Gelenkteils muss das Schlagwerkzeug wegen der Härte des Knochens mit beträchtlicher Kraft betätigt werden. Damit das Schlagwerkzeug sauber, präzis und möglichst ohne Mühe geführt werden kann sind stabile und bequeme Griffe am Schlagwerkzeug von Vorteil. Bei der vorliegenden Erfindung ist vorgesehen, dass mindestens zwei Griffe 3 mit dem Schlagwerkzeug verbunden sind. Dies ergibt einerseits eine gute Stabilität und erlaubt andererseits mit geringem Kraftaufwand eine präzise Führung des Schlagwerkzeugs. Die Griffe können wie in Figur 1 gezeigt an beiden Enden verbunden sein, so dass die Gefahr verringert wird, dass bei einer Manipulation mit dem Schlagwerkzeug etwas daran hängen bleibt oder gar zwischen einen Griff 3 und dem Schlagwerkzeug gelangt wie z.B. ein Schlauch oder ein Stück Stoff. Dies kann zusätzlich noch verbessert werden indem die Griffe 3 bzw. die Befestigung der Griffe 3 am Schlagwerkzeug abgerundet werden.

Mehrere Griffe **3** am Schlagwerkzeug erlauben, das Schlagwerkzeug mit beiden Händen festzuhalten, was für die Stabilität und Präzision noch besser ist. In einer bevorzugten Ausführungsvariante sind zwei Griffe **3** an gegenüberliegenden Seiten der Zylinder/Kolbenanordnung angeordnet. Die beiden Längsachsen G der Griffe definieren die Griffebene (Figur 1a). Drei, vier oder mehr Griffe 3 können auch in regelmässigen Abständen um die Achse **ZK** angeordnet sein. Dies hat den Vorteil, dass der Chirurg während der Bearbeitung des Knochens unterschiedliche Griffe **3** um das Schlagwerkzeug benutzen kann, beispielsweise wenn er seine Arbeitsposition wechseln möchte, ohne das Schlagwerkzeug drehen zu müssen.

Für die gute Handhabbarkeit des Schlagwerkzeugs ist nicht nur die Anzahl der Griffe 3 wesentlich, sondern auch ihre Ausrichtung relativ zum Gehäuse 1. Im Folgenden werden verschiedene vorteilhafte Ausrichtungen der Griffe **3** beschrieben, die die Ergonomie des Schlagwerkzeugs substanziell verbessern.

In einer möglichen Ausführungsvariante der Erfindung können die Längsachse **G** der Griffe **3** und die Längsachse **ZK** der Zylinder/Kolbenanordnung z.B. parallel sein (Figur 1a). Diese Variante ist von Vorteil, wenn der Chirurg das Schlagwerkzeug vertikal vor ihm betätigt, insbesondere auf Brusthöhe.

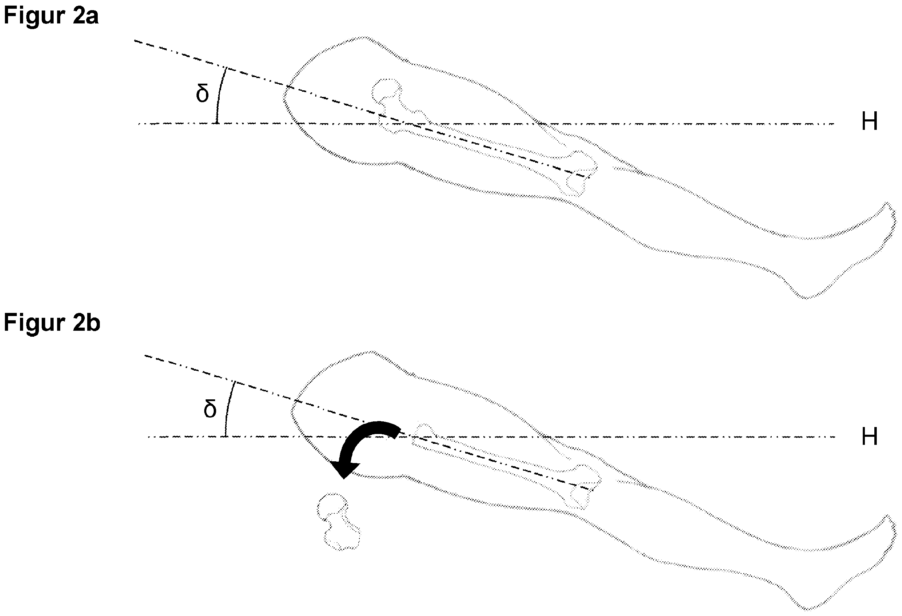

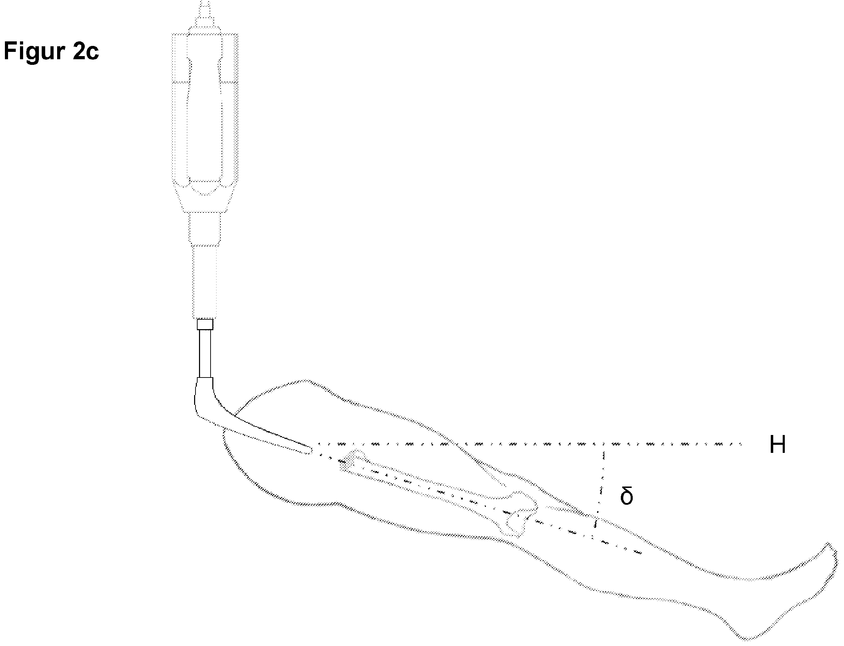

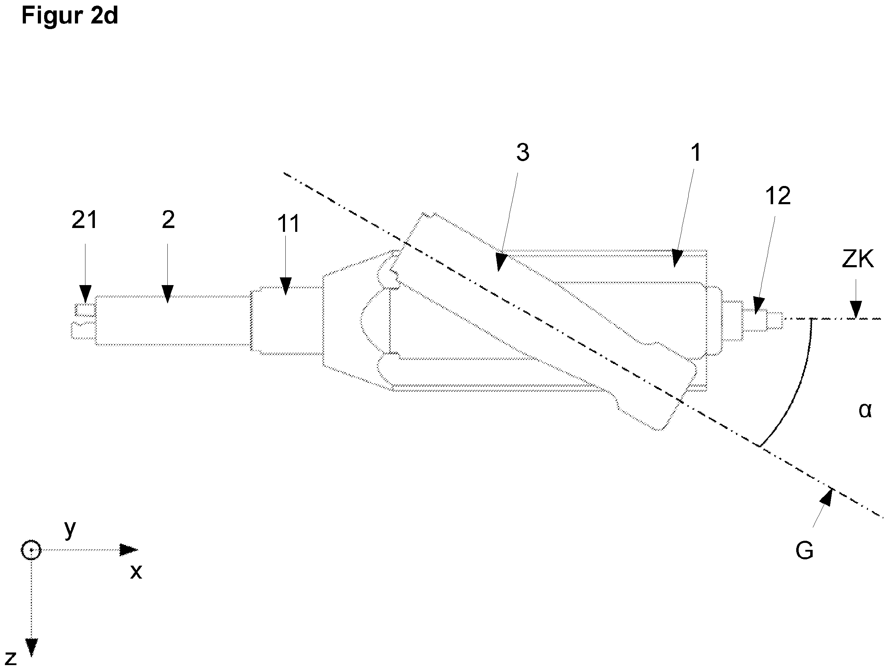

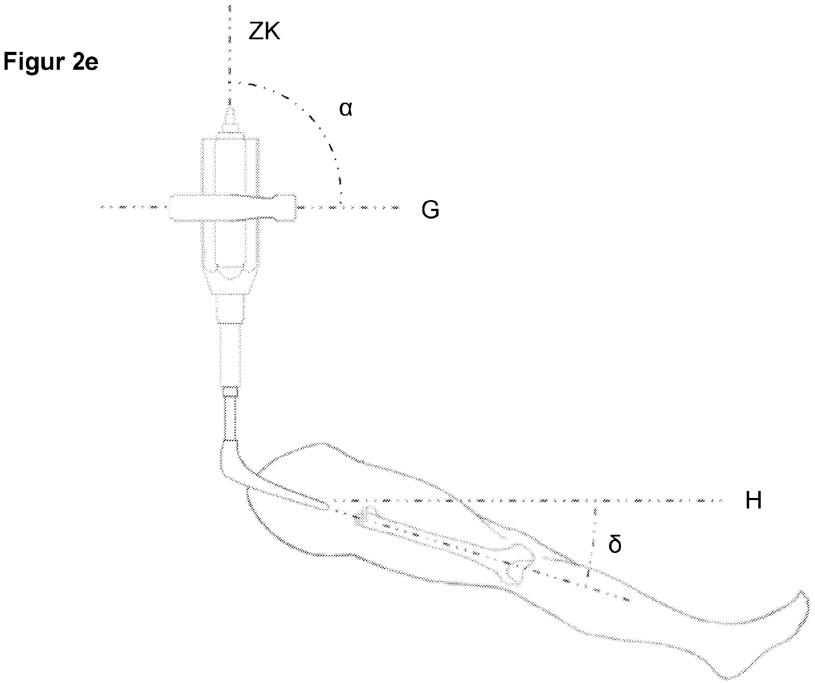

Bei einer Hüftoperation mit der AMIS-Methode liegt der Patient auf dem Rücken. Für die Operation wird das Bein des Patienten nach aussen gedreht und in einem Winkel **δ** von ungefähr 30° nach unten gekippt (Figur 2a-c). Der Knochen für die Aufnahme des künstlichen Gelenkteils wird von danach von Oben her bearbeitet, wobei das Schlagwerkzeug nicht parallel hinter dem Knochen betätigt werden kann, weil der Körper des Patienten im Weg ist. Um dem Chirurgen den Zugang zum Kopf des Oberschenkelknochens zu ermöglichen steht dieser vor dem Patienten und neben dessen Bein. Für die Bearbeitung des Knochens wird ein gebogenes, im wesentlichen bananenförmiges Werkzeug eingesetzt. Dementsprechend wird für eine optimale Bearbeitung des Oberschenkelknochens das Schlagwerkzeug mehr oder weniger vertikal betätigt (Figur 2c). In dieser Arbeitsposition hält der Chirurg das Schlagwerkzeug vertikal vor ihm, ungefähr auf Brusthöhe. Um dem Chirurgen eine optimale Bedienung des Schlagwerkzeugs zu ermöglichen ist denkbar, dass die Griffe 3 um einem Winkel α gedreht werden können (Figur 2d). Dieser Winkel α wird zwischen der Griffebene und der Längsachse **ZK** der Zylinder/Kolbenanordnung gemessen. Da die Griffe 3 lateral in Richtung der Achse y gegenüber der Zylinder/Kolbenanordnung verschoben sind, befinden sich die zwei Achsen **G** und **ZK** nicht in derselben Ebene und können sich somit auch nicht schneiden. Sinnvoll sind Winkel **α** zwischen 0° bis 90°. Neben der Ausführungsvariante mit einem fixen Winkel **α,** könnten die Griffe 3 am Schlagwerkzeug auch so befestigt sein, dass sie gegenüber der Zylinder/Kolbenanordnung gedreht werden können, um den Winkel entweder in Stufen oder stufenlos einstellen zu können. In dieser Art kann das Schlagwerkzeug so ausgeführt werden, dass der Chirurg für jeden Einsatz die beste Griffneigung zur Verfügung hat (Figur 2e).

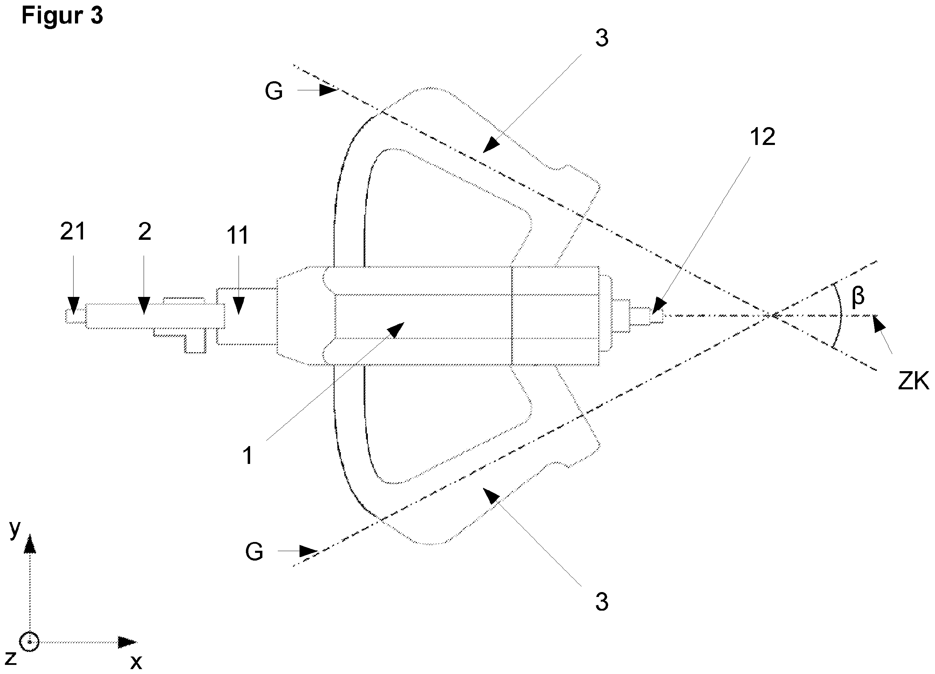

In einer weiteren Ausführungsvariante kann die Längsachse **G** der Griffe 3 auch innerhalb der Griffebene einen Winkel **β** aufweisen (Figur 3). Dieser Winkel **β** trägt zur Ergonomie des Schlagwerkzeugs vorteilhaft bei. Wenn ein Benutzer das Schlagwerkzeug z.B. vertikal vor sich auf Brusthöhe hält, ist sein Griff am Schlagwerkzeug in der Ausführungsvariante der Figur 3 unter Umständen bequemer oder für die Anwendung besser geeignet, als in der Figur 1a gezeigt, wo die Längsachsen der Griffe **3** parallel sind. Ähnlich wie die Position der Hände auf dem Lenkrad eines Autos, d.h. nicht parallel auf entgegengesetzten Seiten des Lenkrads, ist der Griff ergonomischer.

Die Längsachsen **G** der Griffe **3** sind also nicht parallel, sondern bilden einen Winkel **β** mit der Achse **ZK.** Der optimale Winkel **β** kann z.B. je nach den Dimensionen des Schlagwerkzeugs unterschiedlich sein. Der Winkel **β soll** aber **nicht** zu gross sein, damit sich die Achsen **G** und **ZK** nicht innerhalb des Schlagwerkzeugs schneiden, da sonst Einbussen bezüglich der Führungsstabilität zu erwarten sind.

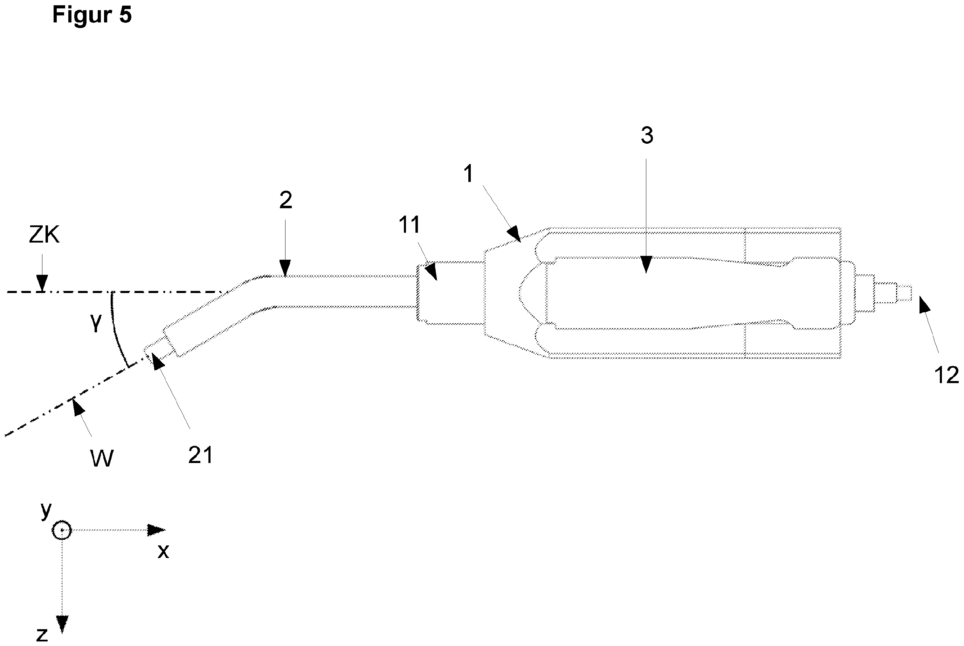

Zusätzlich zur Form und Neigung der Griffe 3 in diversen Richtungen kann auch der Werkzeughalter **2** in unterschiedlicher Weise gestaltet werden. Um mit dem Werkzeug einen besseren Zugang zum Knochen zu erlangen, ist in einer Variante vorgesehen, den Werkzeughalter **2** mit einem Knick zu versehen (Figur 5). Der Knick kann verschiedene Winkel **γ** zwischen 0° und 90° aufweisen, damit der Chirurg das Schlagwerkzeug nicht mit verrenkten Armen und Händen bedienen muss. Dies erlaubt dem Chirurgen, eine ergonomische Arbeitsposition einzunehmen. In Kombination mit den Gestaltungsvarianten für die Griffe 3 kann das Schlagwerkzeug so für jede Operation ergonomisch optimal gestaltet werden.

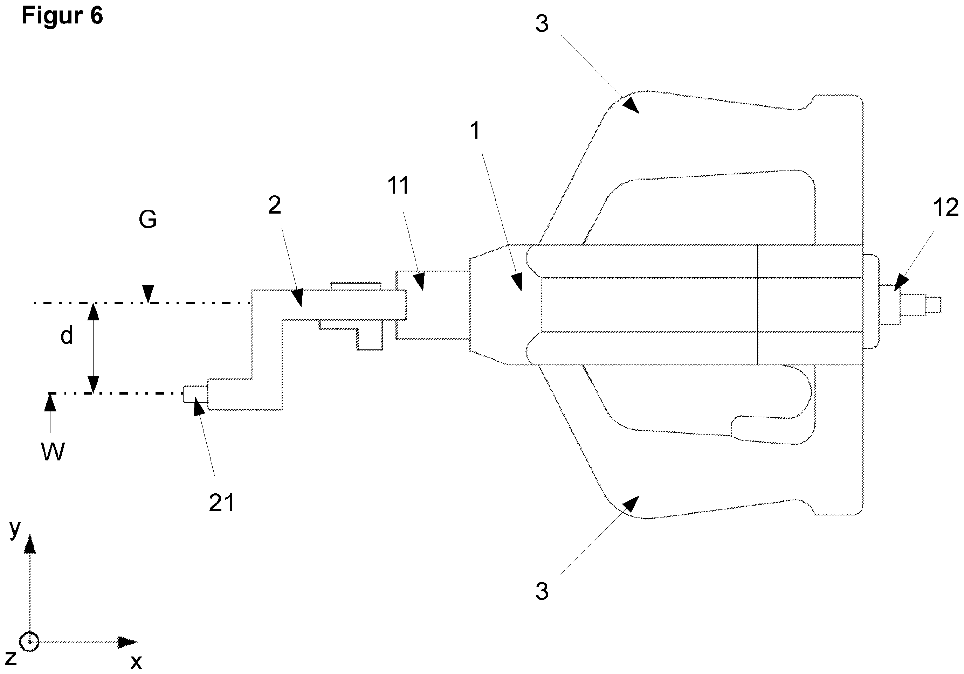

In einer alternativen Variante könnte der Werkzughalter **2** auch einen Versatz **d** aufweisen, wobei die Längsachsen W des Werkzeugs und **ZK** der Zylinder/Kolbenanordnung um einem Abstand **d** verschoben sind (Figur 6). Dieser Abstand d kann auch den Vorteil haben, dass der Chirurg sich weniger nach vorne beugen muss, da das Schlagwerkzeug mit einem Abstand vom Einschnitt gehalten werden kann.

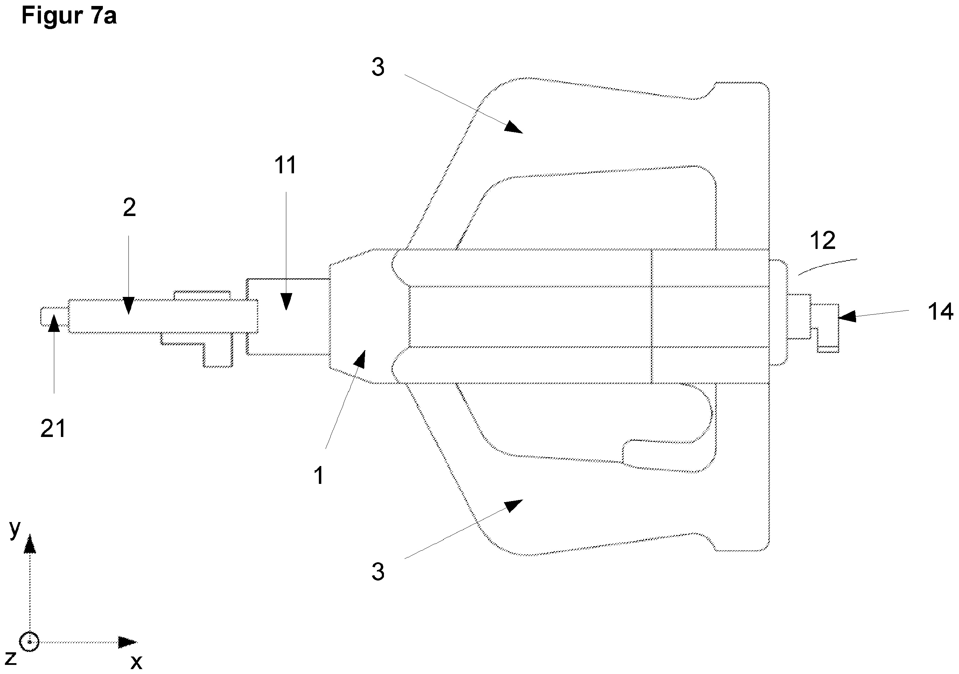

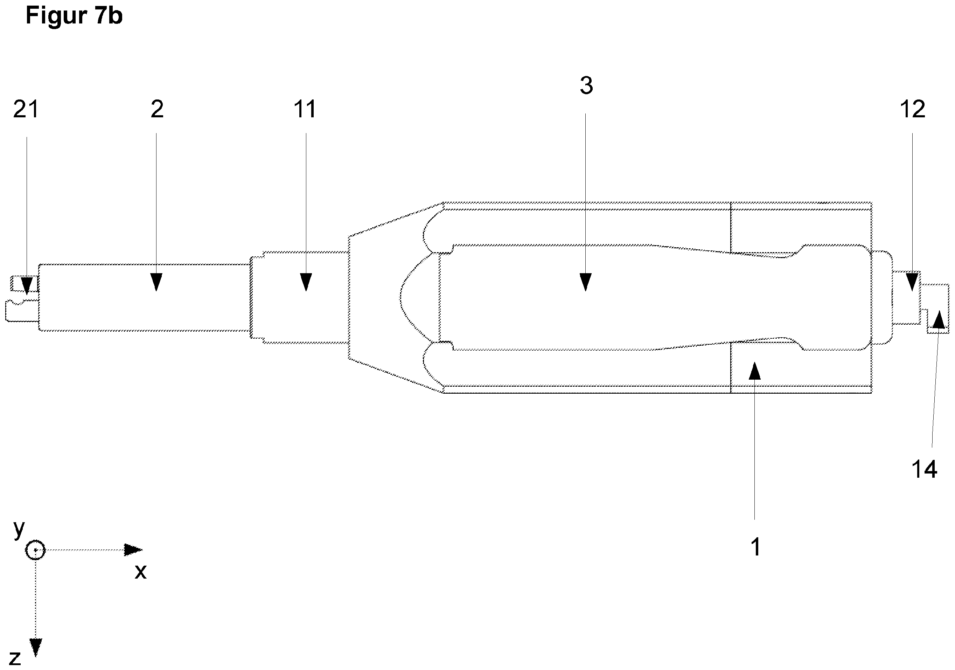

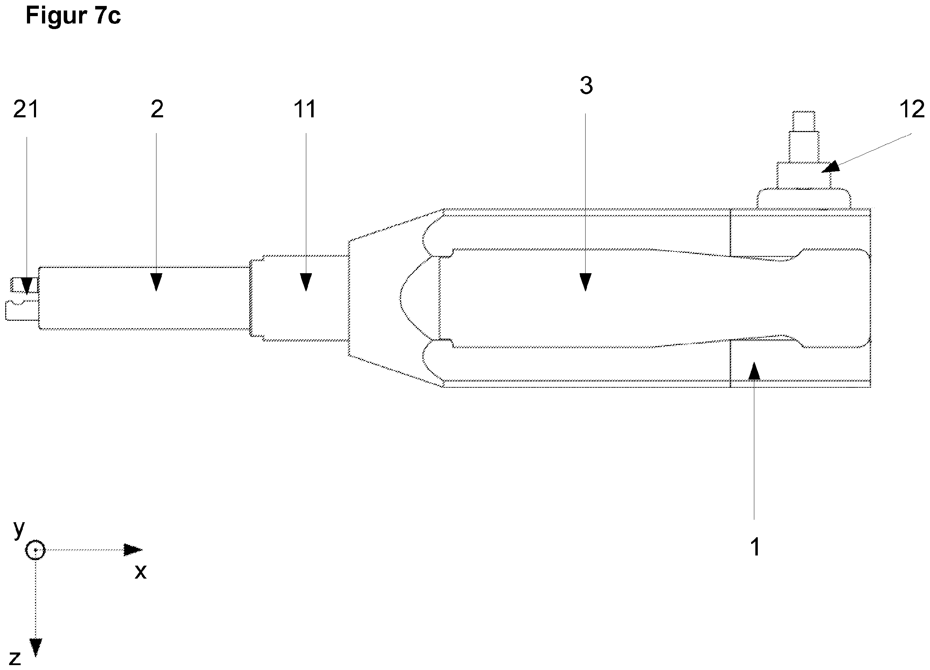

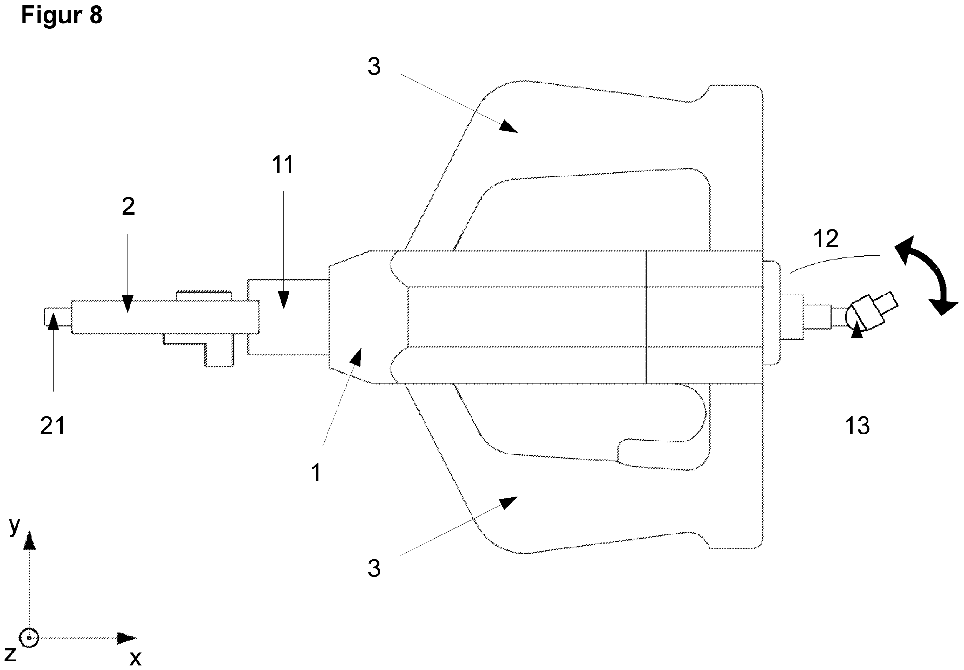

Es ist vorgesehen, die Zylinder/Kolbenanordnung mit Druckluft anzutreiben. Andere Antriebsvarianten, z.B. elektrisch sind aber auch möglich. Die Druckluft kann dem pneumatischen Schlagwerkzeug in einer Variante über einen Schlauch zugeführt werden. Für eine bessere Handhabbarkeit des pneumatischen Schlagwerkzeugs befindet sich der Schlauchanschluss **12** idealerweise am hinteren Ende des pneumatischen Schlagwerkzeugs. Der Anschluss kann gerade nach hinten angeordnet sein (Figur 6) oder einen 90° Winkel quer Längsachse **ZK** der Zylinder/Kolbenanordnung bilden. Befindet sich der Anschluss in der Verlängerung der Längsachse ZK der Zylinder/Kolbenanordnung kann ein Winkelanschluss 14 benutzt werden (Figur 7a und 7b). Ein derartiger Winkelanschluss 14 kann auch um die Längsachse **ZK** der Zylinder/Kolbenanordnung drehbar sein. Alternativ kann der Anschluss aber auch seitlich am Gehäuse angebracht sein (Figur 7c). Um dem benötigten hohen Luftdruck standzuhalten, sind diese Schläuche oft dick, steif und schwer. Damit der Schlauch möglichst wenig stört und keinen Zug auf das Schlagwerkzeug ausübt, weist der Schlauchanschluss **12** in einer Ausführungsvariante ein drehbares Gelenk auf, beispielsweise ein Kugelgelenk **13** (Figur 8). In dieser Ausführungsvariante mit drehbarem Kugelgelenk kann das pneumatische Schlagwerkzeug unabhängig vom Schlauch ohne Widerstand in alle Richtungen gedreht werden.

Noch flexibler ist die Bedienung des pneumatischen Schlagwerkzeugs, wenn gar kein Schlauch nötig ist. Dies kann z.B. durch dass Anbringen einer Druckluftpatrone am Schlagwerkzeug erreicht werden. Um zu vermeiden, dass das Schlagwerkzeug zusammen mit der Druckluftpatrone grösser und damit unhandlicher wird ist vorgesehen, dass die Griffe 3 hohl sein können und die Druckluftpatrone in diesen Hohlraum eingesetzt werden kann. Bei mehreren Griffen **3** können mehrere Patronen verwendet werden, so dass eine grössere Antriebskraft, ein konstanterer Antrieb und/oder eine längere Autonomie erreicht wird.

## Patentansprüche

1. Schlagwerkzeug zur Knochenbearbeitung bei Hüftoperationen, bestehend aus einem Gehäuse (1) mit einer
Zylinder/Kolbenanordnung mit einem pneumatisch oder elektrisch angetriebenen Kolben und einem Werkzeughalter (2), **dadurch gekennzeichnet, dass**
zwei oder mehr Griffe (3) in regelmässigen Abständen um die Zylinder/Kolbenanordnung angeordnet sind.

2. Schlagwerkzeug gemäss Anspruch 1,
**dadurch gekennzeichnet, dass**
die Längsachse (G) des Griffs (3) und die Längsachse (ZK) der Zylinder/Kolbenanordnung im wesentlichen parallel sind.

3. Schlagwerkzeug gemäss Anspruch 1,
**dadurch gekennzeichnet, dass** eine durch die Längsachsen (G) der Griffe 3 definierte Griffebene mit der Längsachse (ZK) der Zylinder/Kolbenanordnung einen Winkel α bildet.

4. Schlagwerkzeug gemäss Anspruch 1,
**dadurch gekennzeichnet, dass** die Längsachsen (G) der Griffe (3) nicht parallel angeordnet sind.

5. Schlagwerkzeug gemäss Anspruch 1,
**dadurch gekennzeichnet, dass**
der Werkzeughalter (2) einen Winkel γ aufweist.

6. Schlagwerkzeug gemäss Anspruch 1,
**dadurch gekennzeichnet, dass**
die Achse (W) des Werkzeughalters (2) gegenüber der Längsachse (ZK) der Zylinder/Kolbenanordnung einen Versatz (d) aufweist.

7. Schlagwerkzeug gemäss Anspruch 1,
**dadurch gekennzeichnet, dass**
der Kolben pneumatisch angetrieben ist und
ein Anschluss (12) für einen pneumatischen Schlauch in der Verlängerung der Längsachse (ZK) der Zylinder/Kolbenanordnung liegt.

8. Schlagwerkzeug gemäss Anspruch 1,
**dadurch gekennzeichnet, dass**
der Kolben pneumatisch angetrieben ist und
ein Anschluss (12) für einen pneumatischen Schlauch senkrecht zur Längsachse (ZK) der Zylinder/Kolbenanordnung liegt.

9. Schlagwerkzeug gemäss Anspruch 1,
**dadurch gekennzeichnet, dass**
der Kolben pneumatisch angetrieben ist und
ein Anschluss (12) für einen pneumatischen Schlauch ein drehbares Gelenk (13) aufweist, und um die Längsachse (ZK) der Zylinder/Kolbenanordnung drehbar ist.

10. Schlagwerkzeug gemäss Anspruch 9,
**dadurch gekennzeichnet, dass**
das Gelenk (13) ein Kugelgelenk ist.

11. Schlagwerkzeug gemäss Anspruch 1,
**dadurch gekennzeichnet, dass**
der Kolben pneumatisch angetrieben ist und mindestens eine Druckluftpatrone mit dem Schlagwerkzeug verbunden oder in das Schlagwerkzeug integriert ist.

12. Schlagwerkzeug gemäss Anspruch 11,
**dadurch gekennzeichnet, dass**
die Druckluftpatrone in den Griffen (3) eingesetzt werden.

## Claims

1. Striking tool for treatment of bones in hip operations,
consisting of a housing (1) with a cylinder/piston arrangement with a pneumatically or electrically driven piston and a tool holder (2),
**characterized in that**
two or more handles (3) are arranged at regular intervals around the cylinder/piston assembly.

2. Striking tool according to claim 1,
**characterized in that**
the longitudinal axis (G) of the handle (3) and the longitudinal axis (ZK) of the cylinder/piston arrangement are essentially parallel.

3. Striking tool according to claim 1,
**characterized in that** a grip plane defined by the longitudinal axes (G) of the grips 3 forms an angle α with the longitudinal axis (ZK) of the cylinder/piston arrangement.

4. Striking tool according to claim 1,
**characterized in that** the longitudinal axes (G) of the handles (3) are not arranged in parallel.

5. Striking tool according to claim 1,
**characterized in that**
the tool holder (2) has an angle γ.

6. Striking tool according to claim 1,
**characterized in that**
the axis (W) of the tool holder (2) has an offset (d) relative to the longitudinal axis (ZK) of the cylinder/piston arrangement.

7. Striking tool according to claim 1,
**characterized in that**
the piston is pneumatically driven and
a connection (12) for a pneumatic hose is in the prolongation of the longitudinal axis (ZK) of the cylinder/piston arrangement.

8. Striking tool according to claim 1,
**characterized in that**
the piston is pneumatically driven and
a connection (12) for a pneumatic hose is perpendicular to the longitudinal axis (ZK) of the cylinder/piston arrangement.

9. Striking tool according to claim 1,
**characterized in that**
the piston is pneumatically driven and
a connection (12) for a pneumatic hose has a rotatable joint (13) and can be rotated about the longitudinal axis (ZK) of the cylinder/piston arrangement.

10. Striking tool according to claim 9,
**characterized in that**
the joint (13) is a ball joint.

11. Striking tool according to claim 1,
**characterized in that**
the piston is pneumatically driven and
at least one compressed air cartridge is connected to the percussion tool or integrated into the percussion tool.

12. Striking tool according to claim 11,
**characterized in that**
the compressed air cartridge can be inserted in the handles (3).

## Revendications

1. Outil de percussion pour le traitement d'os en chirurgie de la hanche, composé d'un boîtier (1) avec un ensemble cylindre/piston avec un piston à entraînement pneumatique ou électrique et un porte-outil (2), **caractérisé en ce que**
deux ou plusieurs poignées (3) sont disposées à intervalles réguliers autour de l'ensemble cylindre/piston.

2. Outil de percussion selon la revendication 1,
**caractérisé en ce que**
l'axe longitudinal (G) de la poignée (3) et l'axe longitudinal (ZK) de l'ensemble cylindre/piston sont sensiblement parallèles.

3. Outil de percussion selon la revendication 1,
**caractérisé en ce qu'** un plan de poignées défini par les axes longitudinaux (G) des poignées 3 forme un angle α avec l'axe longitudinal (ZK) de l'ensemble cylindre/piston.

4. Outil de percussion selon la revendication 1,
**caractérisé en ce que** les axes longitudinaux (G) des poignées (3) ne sont pas disposés parallèlement.

5. Outil de percussion selon la revendication 1,
**caractérisé en ce que**
le porte-outil (2) présente un angle γ.

6. Outil de percussion selon la revendication 1,
**caractérisé en ce que**
l'axe (W) du porte-outil (2) présente un désaxage (d) par rapport à l'axe longitudinal (ZK) de l'ensemble cylindre/piston.

7. Outil de percussion selon la revendication 1,
**caractérisé en ce que**
le piston est actionné pneumatiquement et
un raccord (12) pour un tuyau pneumatique se trouve dans le prolongement de l'axe longitudinal (ZK) de l'ensemble cylindre/piston.

8. Outil de percussion selon la revendication 1,
**caractérisé en ce que**
le piston est actionné pneumatiquement et
un raccord (12) pour un tuyau pneumatique est perpendiculaire à l'axe longitudinal (ZK) de l'ensemble cylindre/piston.

9. Outil de percussion selon la revendication 1,
**caractérisé en ce que**
le piston est actionné pneumatiquement et
un raccord (12) pour un tuyau pneumatique présente une articulation rotative (13) et peut être tourné autour de l'axe longitudinal (ZK) de l'ensemble cylindre/piston.

10. Outil de percussion selon la revendication 9,
**caractérisé en ce que**
l'articulation (13) est une rotule.

11. Outil de percussion selon la revendication 1,
**caractérisé en ce que**
le piston est actionné pneumatiquement et
au moins une cartouche d'air comprimé est reliée à l'outil de percussion ou intégrée à l'outil de percussion.

12. Outil de percussion selon la revendication 11,
**caractérisé en ce que**
la cartouche d'air comprimé est insérée dans les poignées (3).
